# EUROPÄISCHE PATENTSCHRIFT

(11) **EP 1 699 537 B1**
(45) Veröffentlichungstag und Bekanntmachung des Hinweises auf die Patenterteilung: **22.09.2010**
(21) Anmeldenummer: 04804129.7
(22) Anmeldetag: 21.12.2004
(51) Int. Cl.: B01D 3/36, C07D 323/06, B01D 3/14

(54) **VERFAHREN ZUR ABTRENNUNG VON TRIOXAN AUS EINEM TRIOXAN/FORMALDEHYD/WASSER-GEMISCH**
METHOD FOR SEPARATING TRIOXANE FROM A MIXTURE CONTAINING TRIOXANE, FORMALDEHYDE AND WATER
PROCEDE POUR SEPARER LE TRIOXANE D'UN MELANGE DE TRIOXANE/FORMALDEHYDE/EAU

(30) Priorität: 23.12.2003 DE 10361518
(43) Veröffentlichungstag der Anmeldung: 13.09.2006
(73) Patentinhaber: BASF SE, 67056 Ludwigshafen (DE)
(72) Erfinder: LANG, Neven, 68163 Mannheim (DE); STROEFER, Eckhard, 68163 Mannheim (DE); STAMMER, Achim, 67251 Freinsheim (DE); FRIESE, Thorsten, 68163 Mannheim (DE); SIEGERT, Markus, 69126 Heidelberg (DE); OTT, Michael, 69151 Neckargemünd (DE); HASSE, Hans, 67661 Kaiserslautern (DE); GRÜTZNER, Thomas, 70567 Stuttgart (DE); BLAGOV, Sergej, 70195 Stuttgart (DE)
(86) Internationale Anmeldenummer: PCT/EP2004/014531
(87) Internationale Veröffentlichungsnummer: WO 2005/063353

(56) Entgegenhaltungen:
- EP-A- 0 133 669
- DE-A1- 19 526 307
- DE-A1- 19 732 291

## Beschreibung

Die Erfindung betrifft ein Verfahren zur Abtrennung von Trioxan aus einem Trioxan/Formaldehyd/Wasser-Gemisch bei der Herstellung von Trioxan.

Trioxan wird in der Regel durch Destillation von wässriger Formaldehydlösung in Gegenwart saurer Katalysatoren hergestellt. Dem Formaldehyd und Wasser enthaltenden Destillat wird anschließend das Trioxan durch Extraktion mit halogenierten Kohlenwasserstoffen, wie Methylenchlorid oder 1,2-Dichlorethan, oder anderen, mit Wasser nicht mischbaren Lösungsmitteln entzogen.

DE-A 1 668 867 beschreibt ein Verfahren zur Abtrennung von Trioxan aus Wasser, Formaldehyd und Trioxan enthaltenen Gemischen durch Extraktion mit einem organischen Lösungsmittel. Dabei wird eine aus zwei Teilstrecken bestehende Extraktionsstrecke an einem Ende mit einem üblichen organischen, mit Wasser praktisch nicht mischbaren Extraktionsmittel für Trioxan beschickt, am anderen Ende mit Wasser. Zwischen den beiden Teilstrecken wird das zu trennende Destillat der Trioxan-Synthese zuführt. Auf der Seite der Lösungsmittelzuführung wird dann eine wässrige Formaldehydlösung und auf der Seite der Wasserzuführung eine praktisch formaldehydfreie Lösung von Trioxan in dem Lösungsmittel erhalten. In einem Beispiel wird das bei der Trioxan-Synthese entstandene Destillat aus 40 Gew.% Wasser, 35 Gew.% Trioxan und 25 Gew.% Formaldehyd in den Mittelteil einer Pulsationskolonne eindosiert, am oberen Kolonnenende Methylenchlorid und am unteren Kolonnenende Wasser zugeführt. Dabei wird am unteren Kolonnenende eine etwa 25 gew.-%ige Lösung von Trioxan in Methylenchlorid und am oberen Kolonnenende eine etwa 30 gew.-%ige wässrige Formaldehydlösung erhalten.

Nachteil dieser Verfahrensweise ist der Anfall an Extraktionsmittel, welches aufgereinigt werden muss. Bei den verwendeten Extraktionsmitteln handelt es sich zum Teil um Gefahrenstoffe (T oder T⁺-Stoffe im Sinne der deutschen Gefahrenstoffverordnung), deren Handhabung besondere Vorsichtsmaßnahmen erfordert.

DE-A 197 32 291 beschreibt ein Verfahren zur Abtrennung von Trioxan aus einem wässrigen Gemisch, das im Wesentlichen aus Trioxan, Wasser und Formaldehyd besteht, bei dem man dem Gemisch Trioxan durch Pervaporation entzieht und das an Trioxan angereicherte Permeat durch Rektifikation in Trioxan und ein azeotropes Gemisch aus Trioxan, Wasser und Formaldehyd trennt. In dem Beispiel wird ein wässriges Gemisch bestehend aus 40 Gew.-% Trioxan, 40 Gew.-% Wasser und 20 Gew.-% Formaldehyd in einer ersten Destillationskolonne unter Normaldruck in ein Wasser/Formaldehyd-Gemisch und in ein azeotropes Trioxan/Wasser/Formaldehyd-Gemisch getrennt. Das azeotrope Gemisch wird in eine Pervaporationseinheit geleitet, welche eine Membran aus Polydimethylsiloxan mit einem hydrophoben Zeolithen enthält. Das mit Trioxan angereicherte Gemisch wird in einer zweiten Destillationskolonne unter Normaldruck in Trioxan und wiederum in ein azeotropes Gemisch aus Trioxan, Wasser und Formaldehyd aufgetrennt. Dieses azeotrope Gemisch wird vor die Pervaporationsstufe zurückgeführt.

Nachteilig an dieser Verfahrensweise sind die sehr hohen Investitionen für die Pervaporationseinheit.

DE-A-195 26 307 offenbart ein einer Trioxan-Synthesestufe nachgeschaltetes Verfahren zur Abtrennung von Trioxan aus einem Gemisch aus Formaldehyd, Trioxan und Wasser, bei dem das Gemisch in einer ersten Destillationsstufe bei einem Druck von z.B. 0.5 bar destilliert wird, wobei ein erster Strom, der Formaldehyd enthält, und ein zweiter Strom, der überwiegend Trioxan und daneben Wasser und Formaldehyd enthält, erhalten werden. Der zweite Strom wird in einer zweiten Destillationsstute bei einem Druck von z.B. 6 bar destilliert, wobei nahezu reines Trioxan und ein Strom, der überwiegend Trioxan und daneben Wasser und Formaldehyd enthält, erhalten werden.

Aufgabe der Erfindung ist es, ein Verfahren zur Abtrennung von Trioxan aus azeotropen Trioxan/Formaldehyd/Wasser-Gemischen bereitzustellen, welches ohne die Extraktionsschritte oder Pervaporationsschritte des Standes der Technik auskommt.

Gelöst wird die Aufgabe durch ein Verfahren zur Abtrennung von Trioxan aus einem Gemisch aus Formaldehyd, Trioxan und Wasser gemaß Anspruch 1.

Die Gemische enthalten eine Komponente "überwiegend", wenn die betreffende Komponente die Hauptkomponente darstellt, also die Komponente mit dem größeren bzw. größten Massenanteil. Vorzugsweise beträgt der Massenanteil der überwiegend vorliegenden Komponente an dem Gemisch mindestens 50 Gew.-%.

Es ist bekannt, dass Trioxan, Formaldehyd und Wasser ein ternäres Azeotrop bilden, welches bei einem Druck von 1 bar die Zusammensetzung 69,5 Gew.-% Trioxan, 5,4 Gew.-% Formaldehyd und 25,1 Gew.-% Wasser aufweist.

Erfindungsgemäß wird dieses Azeotrop durch Druckwechseldestillation umgangen, bei dem eine erste und eine zweite Destillation bei verschiedenen Drücken durchgeführt werden. In einer ersten Destillationskolonne, welche bei niedrigerem Druck betrieben wird, wird das Ausgangsgemisch in ein Trioxan/Wasser-Gemisch mit geringem Formaldehyd-Gehalt und ein im Wesentlichen trioxanfreies Formaldehyd/Wasser-Gemisch aufgetrennt. Das trioxanfreie Formaldehyd/Wasser-Gemisch kann in die Trioxan-Synthese zurückgeführt werden. In einer zweiten, bei höherem Druck betriebenen Destillationskolonne wird das Trioxan/Formaldehyd/Wasser-Gemisch in reines Trioxan und ein Trioxan/Formaldehyd/Wasser-Gemisch mit niedrigerem Trioxangehalt aufgetrennt.

Als Destillationskolonne sind beliebige Destillationskolonnen wie Packungs- oder Bodenkolonnen geeignet. Die Kolonnen können beliebige Einbauten, Packungen oder Füllkörperschüttungen enthalten.

Der Druck in der zweiten Destillationsstufe ist um mindestens 0,1 bar höher als der Druck in der ersten Destillationsstufe. Im Allgemeinen beträgt diese Druckdifferenz 1 bis 5 bar.

Alle Druckangaben beziehen sich auf den Druck am Kopf der betreffenden Kolonne.

Die erste Destillationsstufe wird bei einem Druck von 0,1 bis 2 bar, vorzugsweise 0,5 bis 2 bar, beispielsweise 1 bar durchgeführt. Die erste Destillationsstufe wird im Allgemeinen in einer Destillationskolonne mit mindestens 2, vorzugsweise 2 bis 50 theoretischen Böden durchgeführt. Im Allgemeinen umfasst der Abtriebsteil mindestens 25 % der Zahl der theoretischen Böden der Kolonne. Vorzugsweise umfasst der Abtriebsteil 50 bis 90 % der theoretischen Stufen der Kolonne. Das Gemisch I, vorzugsweise ein Einspeisungsstrom I, der bei einer vorgelagerten Trioxan-Synthese gewonnen wird, enthält im Allgemeinen über 35 Gew.% Formaldehyd, 25 bis 45 Gew.-% Wasser und 1 bis 30 Gew.-% Trioxan. Dieses Gemisch I wird in einen Strom II, der vorzugsweise am Kolonnensumpf abgezogen wird, und einen Strom III, der vorzugsweise am Kolonnenkopf abgezogen wird, aufgetrennt. Der Strom II enthält im Allgemeinen 51 bis 80 Gew.-% Formaldehyd, 20 bis 49 Gew.-% Wasser und 0 bis 1 Gew.-% Trioxan. Der Strom III enthält im Allgemeinen 1 bis 15 Gew.-% Formaldehyd, 15 bis 35 Gew.-% Wasser und 60 bis 80 Gew.-% Trioxan.

Der Strom II wird in die Trioxan-Synthese zurückgeführt.

Das Gemisch I, welches in der ersten Destillationskolonne destilliert wird, kann auch durch Reaktivdestillation in der ersten Destillationskolonne (die dann als Reaktionskolonne ausgebildet ist) selbst erzeugt werden (siehe unten). In diesem Fall kann der formaldehydhaltige Sumpfabzugsstrom II gering sein und lediglich der Ausschleusung von Hochsiedern dienen. Alternativ dazu kann der Sumpfabzugsstrom II zumindest teilweise in die Reaktionskolonne zurückgeführt werden.

Der Strom III wird mit einem Rückführstrom VII, der in der dritten Destillationsstufe (siehe unten) gewonnen wird, zum Strom IIIa vereinigt. Der Strom IIIa enthält im Allgemeinen 3 bis 20 Gew.-% Formaldehyd, 10 bis 30 Gew.-% Wasser und 60 bis 80 Gew.-% Trioxan.

Die Ströme I, III, IIIa, V und VII können noch bis zu 15 Gew.-% Leichtsieder enthalten. Übliche Leichtsieder, die bei der Trioxan-Synthese und der nachfolgenden destillativen Trennung gebildet werden können, sind Methylformiat, Methylal, Dimethoxydimethylether, Trimethoxydimethylether, Methanol, Ameisensäure sowie weitere Halb- und Vollacetale. Zur Abtrennung dieser Leichtsieder kann optional zwischen der ersten und der zweiten Destillationsstufe eine weitere Destillationsstufe (Leichtsieder-Abtrennstufe) durchgeführt werden. Dabei werden die Leichtsieder vorzugsweise über den Kopf einer Leichtsieder-Abtrennkolonne, welche vorzugsweise bei einem Druck von 1 bis 2 bar betrieben wird, abgetrennt. Im Allgemeinen weist die Leichtsieder-Abtrennkolonne mindestens 5 theoretische Stufen, vorzugsweise 15 bis 50 theoretische Stufen auf. Vorzugsweise umfasst der Abtriebsteil dieser Kolonne 25 bis 90 % der theoretischen Stufen dieser Kolonne. Bevorzugt wird diese Leichtsieder-Abtrennung durchgeführt. Es ist auch möglich, die Leichtsieder bereits aus dem Strom III abzutrennen und anschließend den Strom III mit dem Rückführstrom VII zum Strom IIIa zu vereinigen.

Wird auf eine Leichtsiederabtrennung verzichtet, so fallen die Leichtsieder mit dem Trioxanstrom IV an. Es resultiert dann ein Trioxan geringerer Reinheit.

Der Strom IIIa wird in einer zweiten Destillationsstufe bei einem Druck von 0,2 bis 8 bar in einen Strom IV aus Trioxan und einen Strom V, der überwiegend Trioxan und daneben Wasser und Formaldehyd enthält, aufgetrennt. Diese zweite Destillationsstufe wird bei einem Druck von 0,2 bis 10 bar, vorzugsweise von 2,5 bis 8 bar, beispielsweise bei 4 bar durchgeführt. Im Allgemeinen wird diese zweite Destillationsstufe in einer Destillationskolonne mit mindestens 2 theoretischen Böden, vorzugsweise 5 bis 50 theoretischen Böden, durchgeführt, wobei der Strom IV als Sumpfabzugsstrom oder als Seitenabzugsstrom im Abtriebsteil der Kolonne anfällt und der Strom V als Kopfabzugsstrom anfällt. Im Allgemeinen umfasst der Abtriebsteil der Destillationskolonne 50 bis 90 % der theoretischen Stufen dieser Kolonne.

Im Allgemeinen enthält der Strom IV 95 bis 100 Gew.-%, vorzugsweise 99 bis 100 Gew.-% Trioxan und 0 bis 5 Gew.-%, vorzugsweise 0 bis 1 Gew.-% Wasser und Nebenkomponenten. Nebenkomponenten sind insbesondere die oben genannten Leichtsieder, aber auch höher als Trioxan siedende Komponenten. Besonders bevorzugt ist der Gehalt an Wasser und Nebenkomponenten im Trioxan-Strom IV< 0,1 %. Er kann sogar < 0,01 % sein. Der Strom V enthält im Allgemeinen 5 bis 20 Gew.-% Formaldehyd, 15 bis 35 Gew.-% Wasser und 50 bis 80 Gew.-% Trioxan.

Der Strom V wird in einer dritten Destillationsstufe bei einem Druck von 0,1 bis 4 bar in einen Strom VI, der überwiegend Wasser und daneben Formaldehyd enthält, und den Rückführstrom VII, der überwiegend Trioxan und daneben Wasser und Formaldehyd enthält, aufgetrennt. Vorzugsweise wird die dritte Destillationsstufe bei einem Druck von 0,1 bis 1 bar, beispielsweise 0,2 bar durchgeführt. Im Allgemeinen wird die dritte Destillationsstufe in einer Destillationskolonne mit mindestens einem theoretischen Boden, vorzugsweise 2 bis 20 theoretischen Böden, durchgeführt, wobei der Strom VI als Sumpfabzugsstrom und der Strom VII als Kopfabzugsstrom erhalten werden. Der Abtriebsteil dieser Kolonne umfasst vorzugsweise 40 bis 90 % der theoretischen Stufen dieser Kolonne.

Der Strom VI enthält im Allgemeinen 10 bis 25 Gew.-% Formaldehyd, 75 bis 90 Gew.-% Wasser und 0 bis 1 Gew.-% Trioxan. Der Strom VII enthält im Allgemeinen 5 bis 20 Gew.-% Formaldehyd, 10 bis 30 Gew.-% Wasser und 60 bis 80 Gew.-% Trioxan.

Gegenstand der vorliegenden Erfindung ist auch ein Verfahren zur Herstellung von Trioxan aus einer wässrigen Formaldehydlösung, bei dem der Formaldehyd, Trioxan und Wasser enthaltende Einsatzstrom I in einer vorgelagerten Trioxan-Synthesestufe aus einer wässrigen Formaldehydlösung hergestellt wird und anschließend aus dem Strom I wie vorstehend beschrieben Trioxan abgetrennt wird. Alternativ dazu können die Trioxansynthese und die erste Destillationsstufe in einer Reaktivdestillation vereinigt werden.

In einer Ausführungsform des erfindungsgemäßen Verfahrens wird ein Strom X aus einer wässrigen Formaldehydlösung einer vorgelagerten Trioxan-Synthesestufe zugeführt und in Gegenwart saurer homogen oder heterogen vorliegender Katalysatoren wie Ionenaustauscherharze, Zeolithe, Schwefelsäure und p-Toluolsulfonsäure bei einer Temperatur von im Allgemeinen 70 bis 130 °C umgesetzt. Dabei kann in einer Destillationskolonne oder einem Verdampfer (Reaktiwerdampfer) gearbeitet werden. Das Produktgemisch aus Trioxan/Formaldehyd und Wasser fällt dann als dampfförmiger Brüdenabzugsstrom des Verdampfers bzw. als Kopfabzugsstrom am Kopf der Kolonne an. Die Trioxan-Synthesestufe kann auch in einem Festbett- oder Fließbettreaktor an einem heterogenen Katalysator, z. B. einem Ionenaustauscherharz oder Zeolith, durchgeführt werden.

In einer weiteren Ausführungsform des erfindungsgemäßen Verfahrens werden die Trioxan-Synthesestufe und die erste Destillationsstufe als Reaktivdestillation in einer Reaktionskolonne durchgeführt. Diese kann im Abtriebsteil ein Katalysator-Festbett aus einem heterogenen sauren Katalysator enthalten. Alternativ kann die Reaktivdestillation auch in Gegenwart eines homogenen Katalysators durchgeführt werden, wobei der saure Katalysator zusammen mit der wässrigen Formaldehyd-Lösung im Kolonnensumpf vorliegt.

Im Allgemeinen enthält die wässrige Formaldehydlösung, die der Trioxan-Synthesestufe zugeführt wird, 55 bis 85 Gew.-% Formaldehyd und 15 bis 45 Gew.-% Wasser. Diese Lösung kann in einem vorgelagerten Aufkonzentrierungsschritt aus einer wässrigen Formaldehydlösung mit niedrigerer Formaldehyd-Konzentration erhalten werden. Der Aufkonzentrierungsschritt kann beispielsweise in einem Verdampfer, vorzugsweise einem Fallfilmverdampfer, durchgeführt werden.

Der vorgelagerte Aufkonzentrierungsschritt kann beispielsweise wie in DE-A 199 25 870 beschrieben durchgeführt werden.

Das erhaltene Rein-Trioxan, dessen Reinheit > 99 Gew.-%, > 99,9 Gew.-% oder sogar > 99,99 Gew.-% betragen kann, wird vorzugsweise zur Herstellung von Polyoxymethylen (POM), Polyoxymethylenderivaten wie Polyoxymethylendimethylether (POMDME) und Diaminodiphenylmethan (MDA) verwendet.

Die Erfindung wird nachfolgend mit Bezugnahme auf die Zeichnung näher erläutert.

Es zeigt:

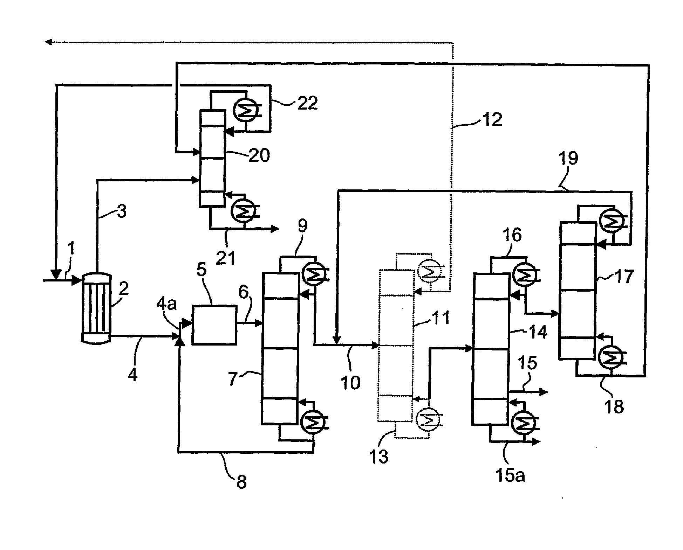

- Figur 1: beispielhaft eine Ausführungsform des erfindungsgemäßen Verfahrens.

Eine wässrige Formaldehydlösung **1** mit einem Formaldehydgehalt von üblicherweise 50 bis 65 Gew.-% wird dem Verdampfer **2,** beispielsweise einem Dünnschichtverdampfer, Fallfilmverdampfer oder Wendelrohrverdampfer zugeführt. Als Brüdenabzugsstrom **3** des Verdampfers wird eine an Formaldehyd abgereicherte wässrige Lösung, als Sumpfabzugsstrom **4** des Verdampfers eine formaldehydreiche wässrige Lösung mit einem Formaldehydgehalt von üblicherweise 55 bis 80 Gew.-% erhalten. Diese wird dem Trioxan-Synthesereaktor **5,** der als Verdampfer, Rührbehälter oder Fest- oder Fließbettreaktor ausgebildet ist, zugeführt. Das den Trioxan-Synthesereaktor verlassende Trioxan/Formaldehyd/Wasser-Gemisch **6** wird der ersten Destillationskolonne **7** zugeführt und dort in einen Formaldehyd/Wasser-Strom **8** (Strom II) und einen Formaldehyd/Wasser/Trioxan-Strom **9** (Strom III) aufgetrennt. Dabei werden der Strom 8 als Sumpfabzugsstrom und der Strom **9** als Kopfabzugsstrom erhalten. Strom **8** wird mit Strom **4** vereinigt und als Strom **4a** in den Reaktor **5** zurückgeführt. Strom **9** wird mit dem Rückführstrom **19** (Strom VII) aus Formaldehyd/Wasser und Trioxan zum Strom **10** (Strom IIIa) vereinigt. Aus dem Strom **10** können in einer Leichtsieder-Abtrennkolonne **11** über Kopf Leichtsieder wie u. a. Methylformiat, Methylal, Dimethoxydimethylether und Methanol als Strom **12** abgetrennt werden. Der Sumpfabzugsstrom **13** wird der Destillationskolonne **14** zugeführt und dort in einen Strom **15** (Strom IV) aus im Wesentlichen reinem Trioxan und einen Strom **16** (Strom V), der überwiegend Trioxan und daneben Wasser und Formaldehyd enthält, aufgetrennt. Strom **15** kann als Seitenabzugsstrom im Abtriebsteil der Kolonne, vorzugsweise gasförmig in der Nähe des Kolonnensumpfs, gewonnen werden. In diesem Fall weist das Trioxan eine besonders hohe Reinheit auf. Als Sumpfabzugsstrom kann ein Strom **15a,** der mit Schwersiedern wie Tetraoxan und weiteren hoch siedenden Nebenkomponenten angereichert ist, gewonnen werden. Der Trioxan-Strom **15** kann auch als Sumpfabzugsstrom gewonnen werden.

Der Strom **16** wird einer dritten Destillationskolonne **17** zugeführt und dort in einen Strom **18** (Strom VI), der überwiegend Wasser und daneben Formaldehyd enthält und den Rückführstrom **19** (Strom VII), der überwiegend Trioxan und daneben Wasser und Formaldehyd enthält, aufgetrennt. Der Strom **18** wird einer weiteren Destillationskolonne **20** zugeführt und dort in einen im Wesentlichen aus Wasser bestehenden Strom **21** und einen Strom **22** aus Formaldehyd-angereicherter wässriger Formaldehydlösung aufgetrennt. In die Kolonne **20** kann auch der Brüdenabzugsstrom **3** des Verdampfers **2** zur Aufkonzentrierung des in diesem enthaltenen Formaldehyds eingespeist werden. Der Formaldehyd/Wasser-Strom **22** wird zusammen mit dem Einsatzstrom **1** in den Verdampfer zurückgeführt.

### Beispiele

Bei der Simulation des in der Figur dargestellten Verfahrens wurden Stoffströme **1, 4a**, **6, 8, 9, 10, 15, 16, 18 und 19** der in den Tabellen angegebenen Zusammensetzungen erhalten. Dabei wurden folgende Parameter angenommen: die erste Destillationsstufe wird bei einem Druck von **1** bar in einer Kolonne **7** mit 16 theoretischen Böden durchgeführt. Das Rücklaufverhältnis beträgt 1,8, die Kopftemperatur 91 °C und die Sumpftemperatur 103 °C. Der Zulauf **6** befindet sich auf Höhe des 4. theoretischen Bodens. Die zweite Destillationsstufe wird bei einem Druck von 4 bar in einer Kolonne **14** mit **8** theoretischen Böden durchgeführt. Das Rücklaufverhältnis beträgt 1, die Kopftemperatur 133 °C und die Temperatur am Seitenabzug **15,** der in Höhe des ersten theoretischen Bodens angebracht ist, 165 °C. Der Zulauf 13 befindet sich auf Höhe des 5. theoretischen Bodens. Die dritte Destillationsstufe wird bei 0,2 bar in einer Kolonne **17** mit 5 theoretischen Böden durchgeführt. Das Rücklaufverhältnis beträgt 0,7, die Kopftemperatur 51 °C und die Sumpftemperatur 62°C. Der Zulauf **16** befindet sich auf Höhe des 3. theoretischen Bodens. Die vierte Destillationsstufe wird bei einem Druck von 4 bar durchgeführt.

### Beispiel 1

| Strom | 1 | 4a (X) | 6 (I) | 8 (II) | 9 (III) | 10 (IIIa) | 15 (IV) | 16 (V) | 18 (VI) | 19 (VII) |
|---|---|---|---|---|---|---|---|---|---|---|
| Mengenstrom [kg/h] | 1408 | 4361 | 4361 | 2952 | 1408 | 8148 | 1000 | 7148 | 408 | 6739 |
| Formaldehyd [Gew.-%] | 0,76 | 0,71 | 0,48 | 0,69 | 0,05 | 0,11 | 0,00 | 0,13 | 0,17 | 0,13 |
| Wasser [Gew.-] | 0,24 | 0,29 | 0,29 | 0,31 | 0,24 | 0,19 | 0,00 | 0,21 | 0,83 | 0,17 |
| Trioxan [Gew.-%] | 0,00 | 0,00 | 0,23 | 0,00 | 0,71 | 0,70 | 1,00 | 0,66 | 0,00 | 0,70 |

### Beispiel 2

| Strom | 1 | 4a (X) | 6 (I) | 8 (II) | 9 (III) | 10 (IIIa) | 15 (IV) | 16 (V) | 18 (VI) | 19 (VII) |
|---|---|---|---|---|---|---|---|---|---|---|
| Mengenstrom [kg/h] | 1456 | 5041 | 5041 | 2952 | 1408 | 8148 | 1000 | 4494 | 456 | 6739 |
| Formaldehyd [Gew.-%] | 0,74 | 0,65 | 0,45 | 0,61 | 0,05 | 0,12 | 0,00 | 0,15 | 0,16 | 0,15 |
| Wasser [Gew.-] | 0,26 | 0,35 | 0,35 | 0,39 | 0,26 | 0,18 | 0,00 | 0,22 | 0,83 | 0,15 |
| Trioxan [Gew.-%] | 0,00 | 0,00 | 0,20 | 0,00 | 0,69 | 0,70 | 1,00 | 0,63 | 0,01 | 0,70 |

### Beispiel 3

| Strom | 1 | 4a (X) | 6 (I) | 8 (II) | 9 (III) | 10 (IIIa) | 15 (IV) | 16 (V) | 18 (VI) | 19 (VII) |
|---|---|---|---|---|---|---|---|---|---|---|
| Mengenstrom [kg/h] | 1493 | 6280 | 6280 | 4787 | 1493 | 5785 | 1000 | 4785 | 493 | 4292 |
| Formaldehyd [Gew.-%] | 0,77 | 0,60 | 0,44 | 0,55 | 0,10 | 0,10 | 0,00 | 0,12 | 0,30 | 0,10 |
| Wasser [Gew.-] | 0,23 | 0,40 | 0,40 | 0,45 | 0,23 | 0,22 | 0,00 | 0,27 | 0,70 | 0,22 |
| Trioxan [Gew.-%] | 0,00 | 0,00 | 0,16 | 0,00 | 0,67 | 0,68 | 1,00 | 0,61 | 0,00 | 0,68 |

### Beispiel 4

| Strom | 1 | 4a (X) | 6 (I) | 8 (II) | 9 (III) | 10 (IIIa) | 15 (IV) | 16 (V) | 18 (VI) | 19 (VII) |
|---|---|---|---|---|---|---|---|---|---|---|
| Mengenstrom [kg/h] | 1449 | 5579 | 5579 | 4129 | 1449 | 4594 | 1000 | 3594 | 449 | 3145 |
| Formaldehyd [Gew.-%] | 0,71 | 0,63 | 0,45 | 0,60 | 0,02 | 0,08 | 0,00 | 0,10 | 0,06 | 0,11 |
| Wasser [Gew.-] | 0,29 | 0,37 | 0,37 | 0,40 | 0,29 | 0,21 | 0,00 | 0,27 | 0,94 | 0,17 |
| Trioxan [Gew.-%] | 0,00 | 0,00 | 0,18 | 0,00 | 0,69 | 0,71 | 1,00 | 0,63 | 0,00 | 0,72 |

## Patentansprüche

1. Verfahren zur Abtrennung von Trioxan aus einem Gemisch I aus Formaldehyd, Trioxan und Wasser, bei dem
a) das Gemisch I in einer ersten Destillationsstufe bei einem Druck von 0,1 bis 2 bar destilliert wird, wobei ein Strom II, der Formaldehyd enthält, und ein Strom III, der überwiegend Trioxan und daneben Wasser und Formaldehyd enthält, erhalten werden,
b) der Strom III mit einem Rückführstrom VII, der überwiegend Trioxan und daneben Wasser und Formaldehyd enthält, gemischt wird, wobei ein Strom IIIa, der überwiegend Trioxan und daneben Wasser und Formaldehyd enthält, erhalten wird,
c) der Strom IIIa, gegebenenfalls nach Abtrennung von Leichtsiedern aus dem Strom III oder IIIa in einer weiteren Destillationsstufe, in einer zweiten Destillationsstufe bei einem Druck von 0,2 bis 10 bar destilliert wird, wobei der Druck in der zweiten Destillationsstufe um mindestens 0,1 bar höher als der Druck in der ersten Destillationsstufe ist, wobei ein Strom IV aus Trioxan und ein Strom V, der überwiegend Trioxan und daneben Wasser und Formaldehyd enthält, erhalten wird,
d) der Strom V in einer dritten Destillationsstufe bei einem Druck von 0,1 bis 4 bar destilliert wird, wobei ein Strom VI, der überwiegend Wasser und daneben Formaldehyd enthält, und der Rückführstrom VII, der überwiegend Trioxan und daneben Wasser und Formaldehyd enthält, erhalten werden,
e) gegebenenfalls der Strom VI in einer vierten Destillationsstufe destilliert wird, wobei ein Strom VIII, der überwiegend Wasser enthält, und ein Strom IX, der überwiegend Formaldehyd enthält, erhalten werden.
wobei das Gemisch I in einer Trioxan-Synthesestufe durch Umsetzung eines Stroms X aus einer wässrigen Formaldehydlösung, der der Trioxan-Synthesestufe zugeführt wird, unter sauren Bedingungen erhalten wird, und wobei der Strom II in die Trioxan-Synthesestufe zurückgeführt wird.

2. Verfahren nach Anspruch 1, **dadurch gekennzeichnet, dass** der Druck in der zweiten Destillationsstufe um 1 bis 5 bar höher als der Druck in der ersten Destillationsstufe ist.

3. Verfahren nach Anspruch 1 oder 2, **dadurch gekennzeichnet, dass** die erste Destillationsstufe bei einem Druck von 0,5 bis 2 bar und die zweite Destillationsstufe bei einem Druck von 2,5 bis 8 bar durchgeführt wird.

4. Verfahren nach einem der Ansprüche 1 bis 3, **dadurch gekennzeichnet, dass** die dritte Destillationsstufe bei einem Druck von 0,1 bis 1 bar durchgeführt wird.

5. Verfahren nach einem der Ansprüche 1 bis 4, **dadurch gekennzeichnet, dass** die erste Destillationsstufe in einer Destillationskolonne mit mindestens 2 theoretischen Böden, die zweite Destillationsstufe in einer Destillationskolonne mit mindestens 2 theoretischen Böden und die dritte Destillationsstufe in einer Destillationskolonne mit mindestens einem theoretischen Boden durchgeführt wird.

6. Verfahren nach einem der Ansprüche 1 bis 5, **gekennzeichnet durch** die nachstehende Zusammensetzung der Ströme I - VII:
| | |
|---|---|
| Strom I: | 35 bis 74 Gew.-% Formaldehyd, 25 bis 45 Gew.-% Wasser, 1 bis 30 Gew.-% Trioxan; |
| Strom II: | 51 bis 80 Gew.-% Formaldehyd, 20 bis 49 Gew.-% Wasser, 0 bis 1 Gew.-% Trioxan; |
| Strom III: | 1 bis 15 Gew.-% Formaldehyd, 15 bis 35 Gew.-% Wasser, 60 bis 80 Gew.-% Trioxan; |
| Strom IIIa: | 3 bis 20 Gew.-% Formaldehyd, 10 bis 30 Gew.-% Wasser, 60 bis 80 Gew.-% Trioxan; |
| Strom IV: | 95 bis 100 Gew.-% Trioxan, 0 bis 5 Gew.-% Wasser und Neben-komponenten; |
| Strom V: | 5 bis 20 Gew.-% Formaldehyd, 15 bis 35 Gew.-% Wasser, 50 bis 80 Gew.-% Trioxan; |
| Strom VI: | 10 bis 25 Gew.-% Formaldehyd, 75 bis 90 Gew.-% Wasser, 0 bis 1 Gew.-% Trioxan; |
| Strom VII: | 5 bis 20 Gew.-% Formaldehyd, 10 bis 30 Gew.-% Wasser, 60 bis 80 Gew.-% Trioxan, |
wobei die Ströme I, III, IIIa, V und VII noch bis zu 15 Gew.-% Leichtsieder, ausgewählt aus der Gruppe bestehend aus Methylformiat, Methylal, Dimethoxydimethylether, Trimethoxydimethylether, Methanol, Ameisensäure sowie weitere Halb- und Vollacetale enthalten können.

7. Verfahren nach einem der Ansprüche 1 bis 6, **dadurch gekennzeichnet, dass** zwischen der ersten und der zweiten Destillationsstufe in einer weiteren Destillationsstufe eine Leichtsiederabtrennung vorgenommen wird.

8. Verfahren nach einem der Ansprüche 1 bis 7, **dadurch gekennzeichnet, dass** der Strom X 55 bis 85 Gew.-% Formaldehyd und 15 bis 45 Gew.-% Wasser enthält.

9. Verfahren nach einem der Ansprüche 1 bis 8, **dadurch gekennzeichnet, dass** der Strom X aus einer wässrigen Formaldehydlösung niedrigerer Formaldehyd-Konzentration durch Aufkonzentrieren in einem Verdampfer erhalten wird.

## Claims

1. A process for removing trioxane from a mixture I of formaldehyde, trioxane and water, by
a) distilling the mixture I in a first distillation stage at a pressure of from 0.1 to 2 bar to obtain a stream II which comprises formaldehyde and a stream III which comprises predominantly trixoane and additionally water and formaldehyde,
b) mixing the stream III with a recycle stream VII which comprises predominantly trioxane and additionally water and formaldehyde to obtain a stream IIIa which comprises predominantly trioxane and additionally water and formaldehyde,
c) distilling the stream IIIa, if appropriate after removing low boilers from the stream III or IIIa in a further distillation stage, in a second distillation stage at a pressure of from 0.2 to 10 bar, the pressure in the second distillation stage being at least 0.1 bar higher than the pressure in the first distillation stage, to obtain a stream IV of trioxane and a stream V which comprises predominantly trioxane and additionally water and formaldehyde,
d) distilling the stream V in a third distillation stage at a pressure of from 0.1 to 4 bar to obtain a stream VI which comprises predominantly water and additionally formaldehyde, and the recycle stream VII which comprises predominantly trioxane and additionally water and formaldehyde,
e) if appropriate, distilling the stream VI in a fourth distillation stage to obtain a stream VIII which comprises predominantly water, and a stream IX which comprises predominantly formaldehyde,
the mixture I being obtained under acidic conditions in a trioxane synthesis stage by converting a stream X of an aqueous formaldehyde solution which is fed to the trioxane synthesis stage, and the stream II being recycled into the trioxane synthesis stage.

2. The process according to claim 1, wherein the pressure in the second distillation stage is from 1 to 5 bar higher than the pressure in the first distillation stage.

3. The process according to claim 1 or 2, wherein the first distillation stage is carried out at a pressure of from 0.5 to 2 bar and the second distillation stage at a pressure of from 2.5 to 8 bar.

4. The process according to any of claims 1 to 3, wherein the third distillation stage is carried out at a pressure of from 0.1 to 1 bar.

5. The process according to any of claims 1 to 4, wherein the first distillation stage is carried out in a distillation column having at least two theoretical plates, the second distillation stage in a distillation column having at least 2 theoretical plates and the third distillation stage in a distillation column having at least one theoretical plate.

6. The process according to any of claims 1 to 5, **characterized by** the following composition of streams I-VII:
| | |
|---|---|
| stream I: | from 35 to 74% by weight of formaldehyde, from 25 to 45% by weight of water, from 1 to 30% by weight of trioxane; |
| stream II: | from 51 to 80% by weight of formaldehyde, 20 to 49% by weight of water, 0 to 1% by weight of trioxane; |
| stream III: | from 1 to 15% by weight of formaldehyde, 15 to 35% by weight of water, 60 to 80% by weight of trioxane; |
| stream IIIa: | from 3 to 20% by weight of formaldehyde, 10 to 30% by weight of water, 60 to 80% by weight of trioxane; |
| stream IV: | from 95 to 100% by weight of trioxane, 0 to 5% by weight of water and secondary components; |
| stream V: | from 5 to 20% by weight of formaldehyde, 15 to 35% by weight of water, 50 to 80% by weight of |
| | trioxane; |
| stream VI: | from 10 to 25% by weight of formaldehyde, 75 to 90% by weight of water, 0 to 1% by weight of trioxane; |
| stream VII: | from 5 to 20% by weight of formaldehyde, 10 to 30% by weight of water, 60 to 80% by weight of trioxane, |
and the streams I, III, IIIa, V and VII may also comprise up to 15% by weight of low boilers selected from the group consisting of methyl formate, methylal, dimethoxydimethyl ether, trimethoxydimethyl ether, methanol, formic acid, and also further hemiacetals and full acetals.

7. The process according to any of claims 1 to 6, wherein a low boiler removal is undertaken in a further distillation stage between the first and the second distillation stage.

8. The process according to any of claims 1 to 7, wherein the stream X comprises from 55 to 85% by weight of formaldehyde and from 15 to 45% by weight of water.

9. The process according to any of claims 1 to 8, wherein the stream X is obtained from an aqueous formaldehyde solution of low formaldehyde concentration by concentrating in an evaporator.

## Revendications

1. Procédé de séparation de trioxane à partir d'un mélange I de formaldéhyde, de trioxane et d'eau, selon lequel
a) le mélange I est distillé lors d'une première étape de distillation à une pression de 0,1 à 2 bar, un courant II, qui contient du formaldéhyde, et un courant III, qui contient principalement du trioxane et également de l'eau et du formaldéhyde, étant obtenus,
b) le courant III est mélangé avec un courant de recyclage VII, qui contient principalement du trioxane et également de l'eau et du formaldéhyde, un courant IIIa, qui contient principalement du trioxane et également de l'eau et du formaldéhyde, étant obtenu,
c) le courant IIIa, éventuellement après séparation de composés de point d'ébullition bas à partir du courant III ou IIIa lors d'une étape de distillation supplémentaire, est distillé lors d'une deuxième étape de distillation à une pression de 0,2 à 10 bar, la pression lors de la deuxième étape de distillation étant supérieure d'au moins 0,1 bar à la pression lors de la première étape de distillation, un courant IV constitué de trioxane et un courant V, qui contient principalement du trioxane et également de l'eau et du formaldéhyde, étant obtenus,
d) le courant V est distillé lors d'une troisième étape de distillation à une pression de 0,1 à 4 bar, un courant VI, qui contient principalement de l'eau et également du formaldéhyde, et le courant de recyclage VII, qui contient principalement du trioxane et également de l'eau et du formaldéhyde, étant obtenus,
e) le courant VI est éventuellement distillé lors d'une quatrième étape de distillation, un courant VIII, qui contient principalement de l'eau, et un courant IX, qui contient principalement du formaldéhyde, étant obtenus,
le mélange I étant obtenu en conditions acides lors d'une étape de synthèse de trioxane par réaction d'un courant X constitué d'une solution aqueuse de formaldéhyde, qui est introduit dans l'étape de synthèse de trioxane, et le courant II étant recyclé dans l'étape de synthèse de trioxane.

2. Procédé selon la revendication 1, **caractérisé en ce que** la pression lors de la deuxième étape de distillation est supérieure de 1 à 5 bar à la pression lors de la première étape de distillation.

3. Procédé selon la revendication 1 ou 2, **caractérisé en ce que** la première étape de distillation est réalisée à une pression de 0,5 à 2 bar et la deuxième étape de distillation à une pression de 2,5 à 8 bar.

4. Procédé selon l'une quelconque des revendications 1 à 3, **caractérisé en ce que** la troisième étape de distillation est réalisée à une pression de 0,1 à 1 bar.

5. Procédé selon l'une quelconque des revendications 1 à 4, **caractérisé en ce que** la première étape de distillation est réalisée dans une colonne de distillation contenant au moins 2 plateaux théoriques, la deuxième étape de distillation dans une colonne de distillation contenant au moins 2 plateaux théoriques et la troisième étape de distillation dans une colonne de distillation contenant au moins un plateau théorique.

6. Procédé selon l'une quelconque des revendications 1 à 5, **caractérisé par** la composition suivante des courants I à VII:
courant I : 35 à 74 % en poids de formaldéhyde, 25 à 45 % en poids d'eau, 1 à 30 % en poids de trioxane ;
courant II : 51 à 80 % en poids de formaldéhyde, 20 à 49 % en poids d'eau, 0 à 1 % en poids de trioxane ;
courant III : 1 à 15 % en poids de formaldéhyde, 15 à 35 % en poids d'eau, 60 à 80 % en poids de trioxane ;
courant IIIa : 3 à 20 % en poids de formaldéhyde, 10 à 30 % en poids d'eau, 60 à 80 % en poids de trioxane ;
courant IV : 95 à 100 % en poids de trioxane, 0 à 5 % en poids d'eau et de composants secondaires ;
courant V : 5 à 20 % en poids de formaldéhyde, 15 à 35 % en poids d'eau, 50 à 80 % en poids de trioxane ;
courant VI : 10 à 25 % en poids de formaldéhyde, 75 à 90 % en poids d'eau, 0 à 1 % en poids de trioxane ;
courant VII : 5 à 20 % en poids de formaldéhyde, 10 à 30 % en poids d'eau, 60 à 80 % en poids de trioxane ;
les courants I, III, IIIa, V et VII pouvant encore contenir jusqu'à 15 % en poids de composés de point d'ébullition bas, choisis dans le groupe constitué par le formiate de méthyle, le méthylal, l'éther diméthoxydiméthylique, l'éther triméthoxydiméthylique,
le méthanol, l'acide formique, ainsi que d'autres semi-acétals et acétals.

7. Procédé selon l'une quelconque des revendications 1 à 6, **caractérisé en ce qu'**une séparation des composés de point d'ébullition bas est réalisée lors d'une étape de distillation supplémentaire entre la première et la deuxième étape de distillation.

8. Procédé selon l'une quelconque des revendications 1 à 7, **caractérisé en ce que** le courant X contient 55 à 85 % en poids de formaldéhyde et 15 à 45 % en poids d'eau.

9. Procédé selon l'une quelconque des revendications 1 à 8, **caractérisé en ce que** le courant X est obtenu à partir d'une solution aqueuse de formaldéhyde de concentration en formaldéhyde plus faible par concentration dans un évaporateur.
